Europäisches Patentamt

European Patent Office

Office européen des brevets

(11) Veröffentlichungsnummer: **0 184 168**
**A2**

(12) # EUROPÄISCHE PATENTANMELDUNG

(21) Anmeldenummer: 85115234.8

(22) Anmeldetag: 30.11.85

(51) Int. Cl.⁴: **A 61 F 5/10**
**A 61 F 5/01**

(30) Priorität: 05.12.84 DE 3444286

(43) Veröffentlichungstag der Anmeldung:
11.06.86 Patentblatt 86/24

(84) Benannte Vertragsstaaten:
AT BE CH DE FR GB IT LI NL SE

(71) Anmelder: Reinhold Hauber Strickwarenfabrik Gmbh & Co. KG
Sigmaringer Strasse 14
D-7440 Nürtingen(DE)

(72) Erfinder: Leuthe, Walter
Hindenburgstrasse 25
D-7440 Nürtingen(DE)

(74) Vertreter: Becker, Maria, Dipl.-Phys.
Auf dem Haigst 29
D-7000 Stuttgart 70(DE)

(54) Gelenkschiene für den Finger einer Hand.

(57) Für einen Finger, der z.B. wegen eines Strecksehnenabrisses nicht mehr aus eigener Kraft gestreckt werden kann, ist eine in sich federnde Gelenkschiene vorgesehen. Eine solche Gelenkschiene besteht aus insgesamt drei wechselseitig an die Handrücken- und Handtellerseite eines Fingers anlegbaren Auflageplatten (1, 2, 3). Diese Auflageplatten sind durch einen Federdraht (7) derart miteinander verbunden, dass der Finger durch die Federkraft des Federdrahtes (7) einerseits in Strecklage gehalten wird und andererseits gegen die Kraft des Federdrahtes (7) aus eigener Kraft gebeugt werden kann.

EP 0 184 168 A2

Dipl.-Phys. M. Decker
Patentanwältin

Auf dem Haigst 29
Telefon (0711) 600306

0184168

26. November 1985

A 6716 / v-nz

Firma Reinhold Hauber,
Strickwarenfabrik GmbH
& Co. KG

Sigmaringer Strasse 14

7440 Nürtingen

_____

Gelenkschiene für den Finger einer Hand.

_____

Die Erfindung betrifft eine Gelenkschiene für den Finger einer
Hand.

Die Aufgabe einer solchen Gelenkschiene soll sein, einen Finger,
der nicht mehr aus eigener Kraft gestreckt werden kann, z.B.
wegen eines Strecksehnenabrisses oder wegen Rheuma, durch
Fremdkraft in die Strecklage zu bringen. Dabei soll die Gelenkschiene ausserdem so aufgebaut sein, dass der geschiente Finger
gegen die Streckkraft der Schiene aus eigener Kraft gebeugt
werden kann.

Gelöst wird diese Aufgabe durch eine Gelenkschiene mit den
Merkmalen nach dem kennzeichnenden Teil des Anspruchs 1.

Zweckmässige Ausgestaltungen der erfindungsgemässen Gelenkschiene sind Gegenstand der Unteransprüche.

Die Wirkungsweise der Gelenkschiene sowie die mit dieser
erzielbaren Vorteile werden im einzelnen anhand des in der
Zeichnung perspektivisch dargestellten Ausführungsbeispiels
beschrieben.

Die Gelenkschiene besteht aus insgesamt drei Auflageplatten
1, 2, 3. Diese Auflageplatten sind jeweils um Achsen 4, 5, 6
schwenkbar. Miteinander verbunden sind die Auflageplatten
1, 2, 3 durch einen an die Achsenden der Auflageplatten
angelenkten Federdraht 7. Der Federdraht 7 bildet bei der
Auflageplatte 3 die Gelenk-Achse 6. Der Anfang und das Ende
des Federdrahtes 7 sind an den Enden der Achse 4 der Auflageplatte 1 angelenkt. Die Anlenkung des Federdrahtes 7 an die
Achse 5 der Auflageplatte 2 erfolgt über eine an den Federdraht
angeformte Windung 8 des Federdrahtes. Dabei ist der Federdraht
jeweils um eine Schraube 10 gewunden, die in Augen 9 der Auflageplatten 1 und 2 eingelassen sind.
Bei in die Gelenkschiene eingeführtem Finger liegt die
Auflageplatte 1 an dem Mittelhandknochen des zu schienenden
Fingers auf dessen Handtellerseite an. Die Auflageplatte 2
liegt an der Handrückenseite an dem an den Mittelhandknochen
angrenzenden Fingerglied an.

Die beiden äussersten Fingerglieder werden von der Auflageplatte 3 wiederum auf der Handtellerseite gestützt.

Der Federdraht 7 ist an den einzelnen Auflageplatten 1 bis 3
so angeformt und in seiner Federwirkung derart ausgelegt,
dass bei eingeführtem Finger dieser in Strecklage gespannt
wird. Bei einem Beugen des Fingers wird der Federdraht 7 im wesentlichen in der Windung 8 in Richtung des Handtellerzentrums verformt, wodurch die Auflageplatte 3 etwa auf einer
Kreisbogenbahn nach innen geführt wird. Bei diesem Bewegungsablauf schwenkt die Auflageplatte 3 um ihre Achse 6. Zwischen
der Auflageplatte 3 und der Fingerspitze ist eine Relativbewegung möglich. Die Lage der Gelenkschiene an dem Finger
wird im wesentlichen durch die Auflageplatten 1 und 2, die
sich nur unwesentlich in Relation zu dem Finger bewegen,
gesichert.

Die Auflageplatten lassen sich am besten aus Kunststoff
formen, um der Anatomie des Fingers optimal angepasst zu
werden.

Während der Federdraht an den Auflageplatten 1 und 2 an an
den Achsenden angeformten Augen 9 angelenkt ist, bildet er
bei der Auflageplatte 3 selbst die Schwenk-Achse 6 der
Auflageplatte.

Mit der dargestellten Gelenkschiene kann ein Finger mit
abgerissener Strecksehne einerseits durch die Federkraft
der Gelenkschiene selbst in Streckstellung gehalten werden
und andererseits gegen die Federkraft problemlos gebeugt
werden.

Die mittlere Auflageplatte 2 kann auch so ausgebildet sein,
dass sie nicht nur an der Handrückenseite an dem betreffenden
Fingerglied anliegt, sondern dieses auf einem grösseren
Umfangsbereich oder sogar vollständig umfasst. Durch ein
stärkeres Umfassen des an den Mittelhandknochen angrenzenden
Fingergliedes ist eine noch wirksamere Fixierung der Gelenkschiene an dem Finger erreichbar. Auch kann mindestens die
Auflageplatte 2 mit einer Beschichtung, z.B. einer Filzauflage,
versehen sein.

Die beschriebene Fingergelenkschiene gewährleistet eine stetige
therapeutische Wirkung, da der ganze Finger von der Handwurzel
bis zum ersten Fingerglied gestreckt gehalten wird. Dennoch ist
eine Fingerbewegung möglich. Durch die längliche Ausbildung
der Auflageplatte 3, die sich über das vordere Fingergelenk
bis zur Fingerkuppe erstreckt, ist eine totale Ruhigstellung
gewährleistet, was bei einem Strecksehnenabriss sehr wichtig
ist. Wenn man die vordere Auflageplatte auf der dem Finger zugewandten Seite muldenartig ausformt, kann der Finger angenehm
gelagert werden.

Durch Verändern der Federspannung, z.B. durch Verbiegen des
Federdrahts, kann eine ständige Korrektur bzw. individuelle
Anpassung an die Anatomie des zu behandelnden Fingers erreicht
werden.

Die Handhabung der Schiene ist äusserst einfach, da sie lediglich auf den Finger aufzuschieben ist. Da wegen der offenen
Gestaltung der Schiene eine gute Belüftung möglich ist, können
Hautreizungen vermieden werden.

*Dipl.-Phys. M. Becker*
*Patentanwältin*

7000 Stuttgart 70
Auf dem Haigst 29
Telefon 04 11 68 08 06

26. November 1985
A 6716 / v-nz

Firma Reinhold Hauber,
Strickwarenfabrik GmbH & Co. KG

Sigmaringer Strasse 14
7440 Nürtingen


P a t e n t a n s p r ü c h e

1. Gelenkschiene für den Finger einer Hand,

g e k e n n z e i c h n e t   d u r c h

folgenden Aufbau:

- Drei Auflageplatten (1, 2, 3) sind jede für sich
  schwenkbar um jeweils getrennte Achsen (4, 5, 6)
  gelagert.

- Die Achsen (4, 5, 6) sind durch ein elastisches
  Verbindungselement (Federdraht 7) miteinander
  verbunden.

- Die Achsen (4, 5, 6) verlaufen in entspanntem Zustand
  der Gelenkschiene in drei übereinanderliegenden Ebenen
  jeweils parallel zueinander.

- In den Achsen (4, 5, 6) der einzelnen Ebenen sind
  angelenkt:

  - in der untersten Ebene die zur Anlage an den
    auf der   Handtellerseite an einen Finger
    angrenzenden Mittelhandknochen bestimmte
    Auflageplatte (1),

  - in der obersten Ebene die zur Anlage an das
    an den Mittelhandknochen angrenzende Fingerglied,
    mindestens an dessen Handrückenseite, bestimmte
    Auflageplatte (2),

- 2 -

0184168

- in der mittleren Ebene die zur Anlage an die
äusseren beiden Fingerglieder auf deren
Handtellerseite bestimmte Auflageplatte (3).

- Der Abstand der die Achsen (4, 5, 6) aufnehmenden
Ebenen ist bei gleichzeitig sich veränderndem
Abstand der in einer senkrecht zu diesen Ebenen
verlaufenden Ebene liegenden Schnittpunkte der
Achsen (4, 5, 6) mit dieser Ebene bei Verformung
des elastischen Verbindungselementes (Federdraht 7)
veränderbar.

- Die elastische Verformbarkeit liegt in der
Beugungsebene des geschienten Fingers.

2. Gelenkschiene nach Anspruch 1, dadurch gekennzeichnet,
dass das elastische Verbindungselement ein an den
Enden der Achsen (4, 5, 6) der Auflageplatten (1, 2, 3)
angelenkter Federdraht (7) ist.

3. Gelenkschiene nach Anspruch 1 oder 2, dadurch gekennzeichnet, dass der Federdraht (7) mindestens eine um
die Achse (5) der obersten Auflageplatte (2) in Beugungsebene der Schiene umlaufende Windung (8) aufweist.

4. Gelenkschiene nach einem der vorhergehenden Ansprüche,
dadurch gekennzeichnet, dass der Federdraht (7) die
Gelenkachse mindestens einer der mittleren oder unteren
Auflageplatte (3, 1) bildet.

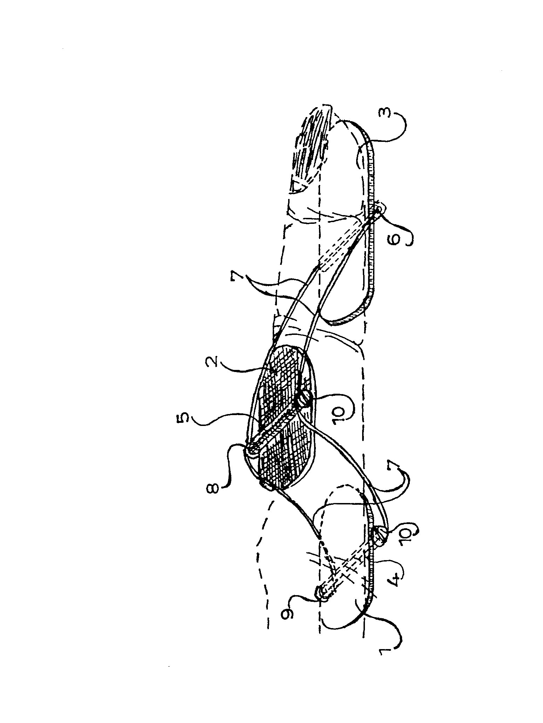

5. Gelenkschiene nach Anspruch 1 bis 3, dadurch gekennzeichnet, dass mindestens die Gelenkachse der obersten
Auflageplatte (2) fest an diese angeformt ist.

0184168